# EUROPEAN PATENT SPECIFICATION

(11) **EP 2 214 739 B1**
(45) Date of publication and mention of the grant of the patent: **28.11.2012**
(21) Application number: 08845781.7
(22) Date of filing: 31.10.2008
(51) Int. Cl.: A61L 31/02, A61L 31/14, A61L 31/16

(54) **ENDOPROSTHESIS WITH POROUS RESERVOIR**
ENDOPROTHESE MIT PORÖSEM RESERVOIR
ENDOPROTHÈSE ÉQUIPÉE D'UN RÉSERVOIR POREUX

(30) Priority: 02.11.2007 US 934421
(43) Date of publication of application: 11.08.2010
(73) Proprietor: Boston Scientific Limited, Christ Church (BB)
(72) Inventor: ATANSOSKA, Liliana, Edina MN 55436 (US); VANCAMP, Daniel, Elk River Minnesota 55330 (US)
(74) Representative: Schley, Jan Malte
(86) International application number: PCT/US2008/082008
(87) International publication number: WO 2009/059146

(56) References cited:
- WO-A1-2009/050168
- US-A1- 2005 119 723
- US-A1- 2006 129 215

## Description

### TECHNICAL FIELD

This disclosure relates to endoprostheses with a porous reservoir.

### BACKGROUND

The body includes various passageways such as arteries, other blood vessels, and other body lumens. These passageways sometimes become occluded or weakened. For example, the passageways can be occluded by a tumor, restricted by plaque, or weakened by an aneurysm. When this occurs, the passageway can be reopened or reinforced with a medical endoprosthesis. An endoprosthesis is typically a tubular member that is placed in a lumen in the body. Examples of endoprostheses include stents, covered stents, and stent-grafts.

Endoprostheses can be delivered inside the body by a catheter that supports the endoprosthesis in a compacted or reduced-size form as the endoprosthesis is transported to a desired site. Upon reaching the site, the endoprosthesis is expanded, e.g., so that it can contact the walls of the lumen. Stent delivery is further discussed in Heath, U.S. 6,290,721.

The expansion mechanism may include forcing the endoprosthesis to expand radially. For example, the expansion mechanism can include the catheter carrying a balloon, which carries a balloon-expandable endoprosthesis. The balloon can be inflated to deform and to fix the expanded endoprosthesis at a predetermined position in contact with the lumen wall. The balloon can then be deflated, and the catheter withdrawn from the lumen.

Passageways containing endoprostheses can become re-occluded. Re-occlusion of such passageways is known as restenosis. It has been observed that certain drugs can inhibit the onset of restenosis when the drug is contained in the endoprosthesis.

US 2006/0129215 A1 discloses implantable or insertable medical devices that contain one or more nanoporous regions, which may further comprise interconnected nanopores. Other aspects of the invention are directed to implantable or insertable medical devices that contain one or more nanostructured regions, which are formed by a variety of methods. Still other aspects of the invention are directed to implantable or insertable medical devices having nanotextured surface regions, in which cell-adhesion-promoting biomolecules (e.g., glycosaminoglycans, proteoglycans, cell adhesion peptides, and adhesive proteins) are provided on, within or beneath the nanotextured surface regions.

US 2005/0119723 A1 discloses an implantable medical device that includes a porous surface with a composite material located within the pores that includes a bioerodable material in combination with a bioactive agent. The composite material is adapted to erode upon exposure to the body of a patient, thus releasing the bioactive agent into the patient, whereas the porous surface remains on the device. In one embodiment, the composite material includes micro- or nanoparticles that are deposited within the pores. In a further embodiment, the porous surface is an electrolessly electrochemically deposited material.

Document WO 2009/050168 A1 discloses a method for the manufacture of a drug eluting nanowire array wherein a polymeric material doped with a therapeutic agent is deposited by electropolymerization within the pores of a nanoporous layer substrate.

### SUMMARY

The present invention relates to a method of forming an endoprosthesis as described in claim 1.

In an aspect, the invention features a method of forming an endoprosthesis, comprising forming a porous metal surface on the endoprosthesis, and introducing a drug into the porous metal surface by applying an electrical potential to the metal surface.

In another aspect, the invention features a method of forming an endoprosthesis, comprising forming a porous metal on the endoprosthesis by selective leaching, and providing a polymer on the porous surface.

In another aspect, the invention features an endoprosthesis comprising a porous metal including a polymer coating conformally within the porous surface.

Embodiments may include one or more of the following features. The drug of the claimed invention is introduced by entrapping the drug in a polymer matrix. The polymer is formed by electropolymerizing. The electrical potential applied to the metal surface can be positive and the monomer can be electrochemically oxidized. The electrical potential applied to the metal surface can be positive and the drug can have a negative charge. The electrical potential applied to the metal surface can be negative and the drug can have a positive charge. The porous metal surface can be formed by dealloying the surface of the endoprosthesis body. The porous metal surface can be formed by etching. The metal surface can be a surface of an endoprosthesis body. An alloy layer can be formed on the endoprosthesis before forming the porous metal surface. The alloy layer can be formed by an implantation process. The porous metal surface can be formed by dealloying the alloy layer. A stent can be formed by forming a porous metal surface on the endoprosthesis and introducing a drug into the porous metal surface by applying an electrical potential to the metal surface.

Embodiments may also include one or more of the following features. A drug can be provided in the polymer. The drug can be provided simultaneously with applying the polymer to the porous surface. The polymer can be applied by electropolymerization. The endoprosthesis can be electrolytically dealloyed. The porous metal can be directly on the surface of a stent body. An alloy layer can be formed on the endoprosthesis before forming the porous metal surface. The alloy layer can be formed by an implantation process. The porous metal surface can be formed by dealloying the alloy layer. A stent can be formed by the process of forming a porous metal on the endoprosthesis by selective leaching, and providing a polymer on the porous surface.

Embodiments may also include one or more of the following features. The polymer coating can include a drug.

Embodiments may include one or more of the following advantages. Stents can be formed with high loadings of drug on select portions, such as the abluminal surface, and the drug can be loaded more readily into the body of the stent, e.g., the porous region of the metal stent surface by applying an electrical potential to the stent. The porous region can have a high porosity, relatively small pore openings, and/or relatively small void cavities which can still be relatively easily infused with drugs facilitated by an applied electrical potential and at the same time can accommodate a sufficient amount of drug. The drug can be delivered to the porous metal region with an electropolymerizable monomer while the metal stent acts as a working electrode of the electropolymerization process. The conformal coating can reduce corrosion of the porous surface and can also include a drug. For example, during electropolymerization a drug can be entrapped in the polymer film or polymer matrix grown in the voids or pores of the metal stent surface regions that formed from the monomer. The drug can also be loaded to the porous region by rendering the drug charged, e.g., forming charged polymer-drug conjugate. The porous region also provides rough surface and allows more surface areas and thus can enhance adhesion of the polymer to the stent. The porosity of the metal surface region can be carefully controlled, e.g. the pore size can be controlled by selecting alloy compositions or the concentration of sacrificial components embedded in a metal stent surface without forming an alloy, and/or by tuning etching (e.g., dealloying) conditions such that a desired porous structure can be obtained to accommodate a sufficient amount of drug or to accommodate a drug-containing polymer carrier or matrix to reduce the likelihood of polymer delamination and protects the substances (e.g., the drug or drug-containing polymer) during delivery of the device into the body. The porous surface region can be highly porous for accommodating a large quantity of drug and at the same time relatively thin, so as not to degrade the performance of the stent

Still further aspects, features, embodiments, and advantages follow.

### DESCRIPTION OF DRAWINGS

FIGS. 1A-1C are longitudinal cross-sectional views illustrating delivery of a stent in a collapsed state, expansion of the stent, and deployment of the stent, respectively.
FIG. 2 is a perspective view of a stent.
FIG. 3 is a cross-sectional view of region of a stent wall while FIG. 3A is a greatly enlarged view of region 3A of FIG. 3.
FIGS. 4A-4C are cross-sectional views illustrating a method for forming a stent.
FIG. 5 is a flow diagram illustrating another manufacture method of a stent.
FIGS. 6A-6C are schematics of an ion bombardment system.

### DETAILED DESCRIPTION

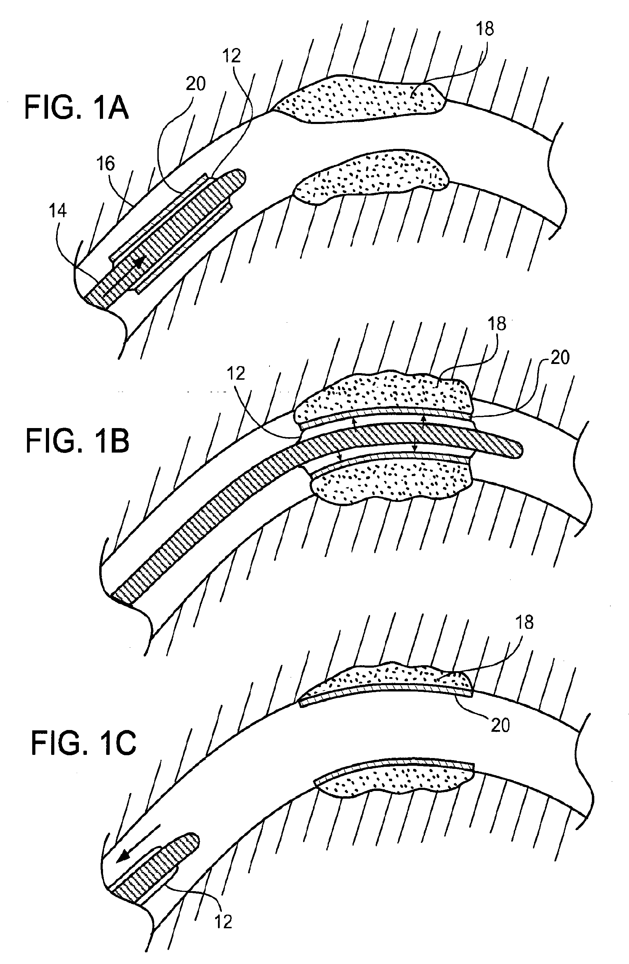

Referring to FIGS. 1A-1C, a stent 20 is placed over a balloon 12 carried near a distal end of a catheter 14, and is directed through the lumen 16 (FIG. 1A) until the portion carrying the balloon and stent reaches the region of an occlusion 18. The stent 20 is then radially expanded by inflating the balloon 12 and compressed against the vessel wall with the result that occlusion 18 is compressed, and the vessel wall surrounding it undergoes a radial expansion (FIG. 1B). The pressure is then released from the balloon and the catheter is withdrawn from the vessel (FIG. 1C).

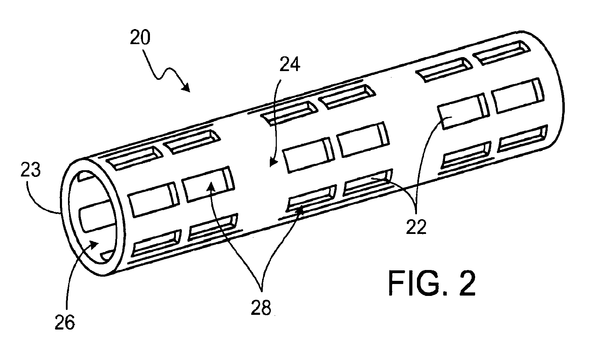

Referring to FIG. 2, the stent 20 includes a plurality of fenestrations 22 defined in a wall 23. Stent 20 includes several surface regions, including an outer, or abluminal, surface 24, an inner, abluminal, surface 26, and a plurality of cutface surfaces 28. The stent can be balloon expandable, as illustrated above, or a self-expanding stent. Examples of stents are described in Heath '721, supra.

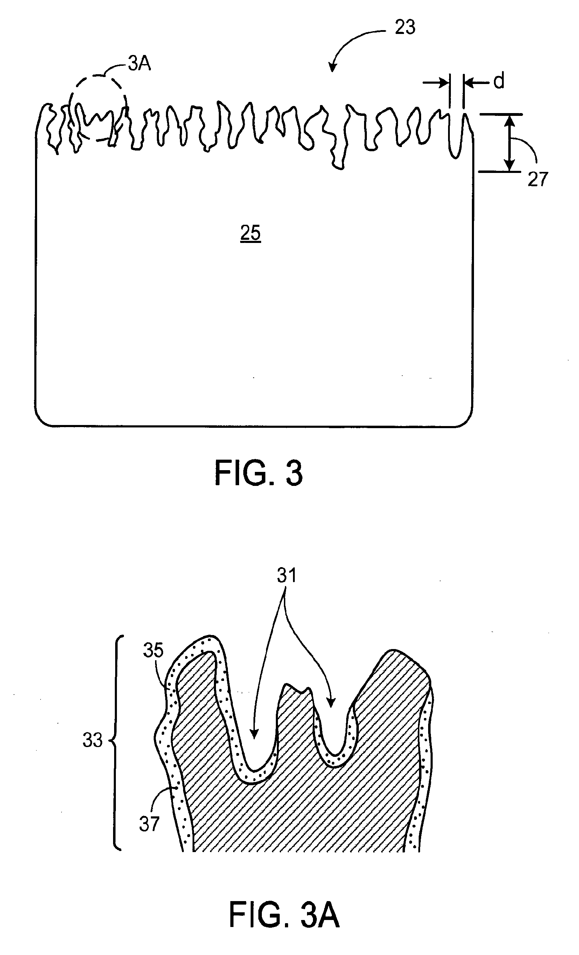

Referring to FIG. 3, a cross-sectional view, a stent wall 23 includes a stent body 25 formed, e.g. of a metal, and includes a porous region 27 on the abluminal side, which can be an integral surface portion of the sent body 25. Referring to Fig. 3A, the porous region has pores or voids 31 in which a composition 33 is stored. The composition 33 can be formed of polymer carrier or matrix 35 (shown as enclosed blank areas) and drug 37 (shown as dots). The composition can be deposited conformally or non-conformally over the porous region 27. For example, a conformal coating formed of the composition 33 can conform to substantially any shape of the porous region, including crevices, points, sharp edges, and exposed internal surfaces, e.g., surfaces that define voids 31, without filling the pores. In other embodiments, the coating fills the pores.

The porous region can be formed with high porosity which can accommodate large volumes of drug and small pore openings which can reduce the likelihood of premature release of excessive doses of drug by, e.g., diminishing drug diffusion to the surrounding environment before stent is deployed. Moreover, the high porosity can allow the porous region to be relatively thin without substantially degrading the stent mechanical performance. In embodiments, the porous region is formed directly in the outer surface of a stent body, e.g. of stainless steel, without depositing a separate reservoir layer over the body. In embodiments, the porous region has an average depth of about a few nanometers ("nm") to a few hundred nanometers, e.g., of about 10nm to 500nm, or about 10-350 nm, or about 10-20nm. In embodiments, the pore size d is selected to be about 1-100 nm in pore diameter. In particular embodiments, the porosity (the ratio of the void volume to total volume of solid and void) of the porous region is about 30%, or more, e.g. about 50% or more, e.g. about 60%.

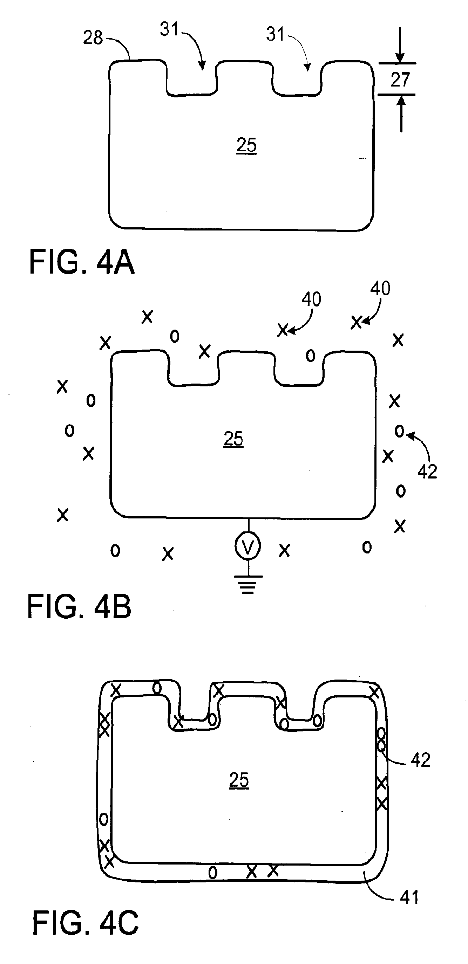

The composition coating, e.g., a drug-containing polymer is selected for compatibility for the porous region and to have a controlled drug elution and therapeutic properties. In embodiments, the composition has a thickness of about 1 to about 1000 nm, e.g., about 10 to about 100 nm. In particular embodiments, substantially all of the composition coating 33 is housed within the voids 31, e.g., 70% or more (e.g., 80% or more, or 90% or more) of the composition coating is formed within the voids, or in other words, less than 30% (e.g., less than 20%, or less than 10%) of the composition is exposed and unprotected by the porous region, which may be more prone to delamination and/or premature drug-release. In further embodiments, due to the formation of composition coating within voids as discussed above, drug to be released first gets eluted from the polymer matrix into the porous region then diffuses from the porous regions into body, thus the drug-release profile can be controlled not only by selecting the polymer matrix but also by tuning the void dimensions, e.g., void depth, void diameter or width. Referring to FIGS. 4A-4C, enlarged cross-sectional side views of a portion of a stent wall illustrate exemplary procedures of forming a stent. Referring particularly to FIG. 4A, the stent wall includes a body 25 formed, e.g. of a metal such as a stainless steel alloy. The stent body includes surface 28, which may be any or all of the abluminal, adluminal or cut-face surfaces, and porous region 27 in surface 28 which defines a plurality of pores or voids 31.

In embodiments, the porous region 27 can be an integral portion of the stent body 25 and can be formed by etching stent surface, e.g., selective leaching or other techniques such as laser ablation. Selective leaching, such as dealloying, demetalification, or parting, is a corrosion type in alloys, when in suitable conditions a component of the alloy is preferentially leached from the material. The more electrochemically active component, usually a less noble metal, is selectively removed from the alloy by microscopic-scale galvanic corrosion mechanism, resulting in the formation of a porous sponge composed substantially almost entirely of the more noble alloy constituents, with usually nanometer-sized pores. The more susceptible alloys are the ones containing elements with longer distance between each other in the galvanic series, e.g., copper and zinc in brass. An alloy may include any suitable combination of metals or combination of a metal and a non-metal, such as carbon and silicon. Dealloying process may include dissolving one or more components of the alloy in a caustic substance. For example, a stainless steel alloy, or aluminum alloy can be dealloyed in sodium hydroxide solution while a zinc alloy such as brass can be dealloyed in an acidic solution. Typically, one or more of the most electrochemically active components of the alloy are dissolved. In certain embodiments, dealloying process can be facilitated by applying electrical potential to the alloy in the caustic substance, e.g., by using an electrolytic cell. The structural morphologies of the porous region such as porosity, pore size and pore depth can be selected by controlling dealloying conditions, such as concentration of the caustic substance, pH value, reaction temperature, electrical potential applied, and processing time. In general, higher reaction temperature and/or longer processing time produces higher porosity and larger pore size. The structural morphologies of the porous region can also be selected by controlling alloy composition in the surface region, as will be described further below. The potential can be cycled or at a fixed potential. The cycling of the potential can be between positive or negative values or within a positive or negative regime. A fixed potential can be positive or negative. Dealloying and other techniques are further described in Deakin et al., Corrosion Science, 46, 2117-2133 (2004), Senior et al., Nanotechnology, 17, 2311-2316 (2006), Bayoumi et al., Electrochemistry Communications 8, 38-44 (2006), and Greely et al., Electrochimica Acta (2007) 5829-5836. Selective etching is also discussed in U.S. Application No. 11/934,296, filed November 2, 2007 [Attorney Docket No. 10527-816001].

Other selective etching techniques can be utilized when the surface 28 of stent body 25 is modified by, e.g., embedding some sacrificial components such as particles of less noble metal in the surface without forming an alloy of the sacrificial metal and the stent metal. The sacrificial metal particles are then selectively removed or etched. The structural morphologies of the porous region can be selected by controlling the concentration of non-alloying sacrificial components embedded in the surface region. In certain embodiments, the porous region can include a ceramic, e.g., titania ("TiOx"), or alumina. In a particular embodiment, the porous region includes titania nanotubes formed by dealloying and anodic oxidation, as discussed in detail by Bayoumi et al., Electrochemistry Communication, 8, 38-44 (2006). In other particular embodiments, a pure metal surface (e.g., not an alloy) can be selectively dissolved to form a porous surface region. For example, an electropolymerized polypyrrole film can be formed in parallel with the formation of a porous alumina layer on an aluminum surface, as described in Liu et al., J. Braz. Chem. Soc., 18, 143-152 (2007).

Referring particularly to FIG. 4B, after the porous region has been formed, the stent is immersed into an electrolyte including an electropolymerizable monomer 40 (shown as crosses) and a therapeutic agent or drug 42 (shown as open circles) in an electrolytic cell, where the stent body 25 is utilized as a working electrode. Although not shown in FIG. 4B, a counter electrode and/or a reference electrode can be included in the cell. Referring to FIG. 4C, the therapeutic agent 42 can be entrapped in a polymer matrix 41 which grows onto the working electrode surface from the electrolyte containing the monomer 40 and the therapeutic agent 42.

In embodiments, during the electropolymerization process, stent body 25 as the working electrode is given a positive potential, e.g., about a few hundred millivolts to about a few volts, and monomer 40, e.g., pyrrole, is electrochemically oxidized at a polymerization potential giving rise to free radicals. In other embodiments, the stent as the working electrode is provided with a negative potential, e.g., about -100 millivolts to about -3 volts, and monomer 40, e.g., 4-vinylpyridine, is electrochemically reduced and is giving rise to free radicals. These radicals are adsorbed onto or chemically bonded to the electrode surface and undergo subsequently a wide variety of reactions leading to the polymer network. The electropolymerization should preferably occur in a solution compatible for the drug to be incorporated into the polymer film in a suitable form. For example, organic solvents such as acetone, acetonitrile, tetrahydrofuran ("THF"), dimthyl formamide ("DMF"), and dimethylsulfoxide ("DMSO"), which can dissolve both the monomer and the drug, are suitable for producing the solution in which electropolymerization occurs. The growth of the corresponding polymer depends on its electrical character. If the polymer is electrically non-conducting, its growth is self-limited. Such films are very thin (about 10 - 100 nm). In contrast, the growth of conductive polymers is virtually unlimited. In the latter case, the growth process is governed by the electrode potential and by the reaction time, which allows control of the thickness of the resulting film. The polymerization occurs locally and strictly on the electrode surface and the drug is entrapped in close proximity to the electrode surface. In addition, the combination of different conducting or non-conducting polymers allows the building of multilayer structures with extremely low thickness leading to different drug release profiles. The polymer film or coating can be generated by cycling the potential ("potentiodynamically") or at a fixed potential ("potentiostatically"). The latter may allow enhanced control of the film thickness and its growth. The cycling of the potential can be between positive or negative values or within a positive or negative regime. A fixed potential can be positive or negative.

In embodiments, the morphology of the polymer film or coating can be selected by controlling the nature of the electrolyte, the crystallographic structure of the underlying electrode, the speed and the potential of the deposition, the presence of counter ions such as anions and polyanions when monomer is electrochemically oxidized or surfactants, the concentration of the monomer, and the pH of the solution. In embodiments, the counter ion can include the drug, such as a polyanion-drug conjugate or charged drug molecules. In some embodiments, the drug is ionically bound and/or covalently bonded to the polymer film. In some embodiments, the drug is either trapped in the polymer matrix or bound to the film surface. In some embodiments, electropolymerized films formed of hybrid materials, e.g., organic-inorganic hybrid materials can be grown on the electrode surface by introducing metal-complex counter ions, such as [Au(CN)₂]⁻, PMo₁₂O₄₀³⁻, and [M(dmit)₂]ⁿ⁻ where M can be Ni, Pd, or Sn and n is the number of charges. In still some embodiments, nanoparticles such as drug nanocrytals or metal nanocrystals can be entrapped in the polymer film during electropolymerization, in which case choices of electrolyte solvent are not necessarily limited to solvents that can dissolve the drug and thus more options of drugs to be enclosed in the polymer can be selected. Examples of polymers obtainable from electropolymerization include but are not limited to conductive polymers such as polypyrrole (PPy) or polythiophene; amine-based polymers such as polyethyleneimine (PEI), polypropyleneimine (PPI), and poly(p-phenylenediamine) (PPPD); poly(N-vinylcarbazole) (PVK); polyphenol; polyvinylpyridine, and their derivatives. Different biological components have been immobilized into electropolymerized films, ranging from whole cells to protein fragments. Electropolymerization is further disclosed in Atanasoska, Chem. Mater., 4, 988-994 (1992); Lakard et al., Bioelectrochemistry, 62, 19-27 (2004); Kowalski et al., Corrosion Science, 49, 1635-1644 (2007); Reyna-Gonzalez et al., Polymer, 47, 6664-6672 (2006); da Cruz et al., Electrochimica Acta, 52, 1899-1909 (2007), and Deniau et al., Journal of Electroanalytical Chemistry, 505, 33-43 (2003), Bak et al., Electrochemistry Communications 7 (2005) 1098-1104. More examples of suitable polymers and drugs are disclosed in Miller et al., US Patent No. 4,585,652.

In embodiments, only the porous region of the stent is coated with drug and/or polymer by exposing only the porous region to the electrolyte. For example, other surface areas can be selectively masked before the stent is immersed into the electrolyte, or only the surface having the porous region is immersed in the electrolyte while the others are not, or after the electrochemical deposition, coatings on the other surfaces are removed by, e.g., grinding, or laser ablation. In embodiments, different portions of the porous region can be loaded with different drugs and/or polymers by, e.g., masking the desired portions of the porous region so that drug will not be deposited in the masked portions of the porous region in a first electropolymerization process, and then removing the mask of those potions so that the drug or a different drug will be deposition in them in a second electropolymerization process. As a result, more diverse drug release profiles can be achieved.

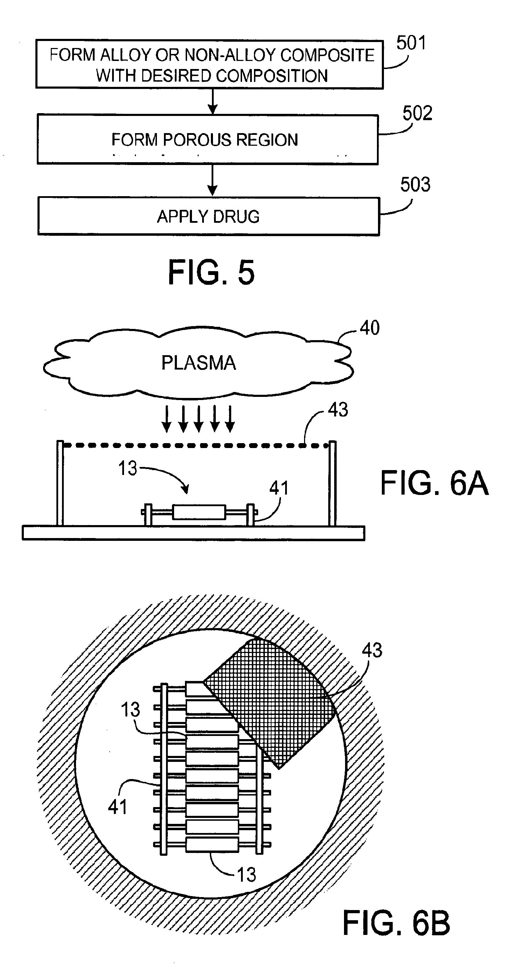

Referring to FIG. 5, an endoprosthesis, e.g., stent is formed by first providing an alloy or a non-alloy composite with desired composition in the surface region of the stent (step 501). Next, a porous region is formed by, e.g., dealloying the alloy or selectively etching the composite (step 502). Finally, a drug is delivered into the voids of the porous region (step 503).

As discussed above, the structural morphologies of the porous region such as porosity, pore size, and pore depth can also be selected by controlling alloy composition or the concentration of non-alloying sacrificial components embedded in the surface region of a stent formed, e.g., of a metal. Referring particularly to step 501 in FIG. 5, in some embodiments, alloy or non-alloy composite with selected composition can be formed by implanting the surface region of the stent with sacrificial elements or components and the alloy or composite can be an integral surface portion of the sent body. There are many techniques to implant a metal substrate, such as plasma immersion ion implantation ("PIII") and laser surface processing. For example, magnesium, aluminum, zinc, or other electrochemically more active metal as sacrificial elements can be implanted or embedded in a stent formed of stainless steel by metal plasma immersion ion implantation and deposition ("MPIIID"). In other embodiments, an alloy overlayer on stent body can be formed by, e.g., physical vapor deposition ("PVD") such as sputtering and pulsed laser deposition ("PLD").

Referring to Figs 6A-6C, the alloy or composite with selected composition can be formed, e.g., using an ion implantation process, such as plasma immersion ion implantation ("PIII"). Referring to FIGS. 6A and 6B, during PIII, charged species in a plasma 40, such as a metal plasma, are accelerated at high velocity towards stents 13, which are positioned on a sample holder 41. Metal plasmas can be produced by vacuum arcs. Acceleration of the charged species of the plasma towards the stents is driven by an electrical potential difference between the plasma and an electrode under the stent. Upon impact with a stent, the charged species or ions, due to their high velocity, penetrate a distance into the stent and implant ions to the material of the stent, forming alloy or composite in the surface region as discussed above. Generally, the porosity of the porous region subsequently formed is selected by controlling composition of the alloy or the composite as formed, e.g., by controlling the concentration of the sacrificial elements or components. And the pore depth can be controlled by ion penetration depth, which is controlled, at least in part, by the potential difference between the plasma and the electrode under the stents. If desired, an additional electrode, e.g., in the form of a metal grid 43 positioned above the sample holder, can be utilized. Such a metal grid can be advantageous to prevent direct contact of the stents with the radio frequency ("RF") plasma between high-voltage pulses and can reduce charging effects of the stent material.

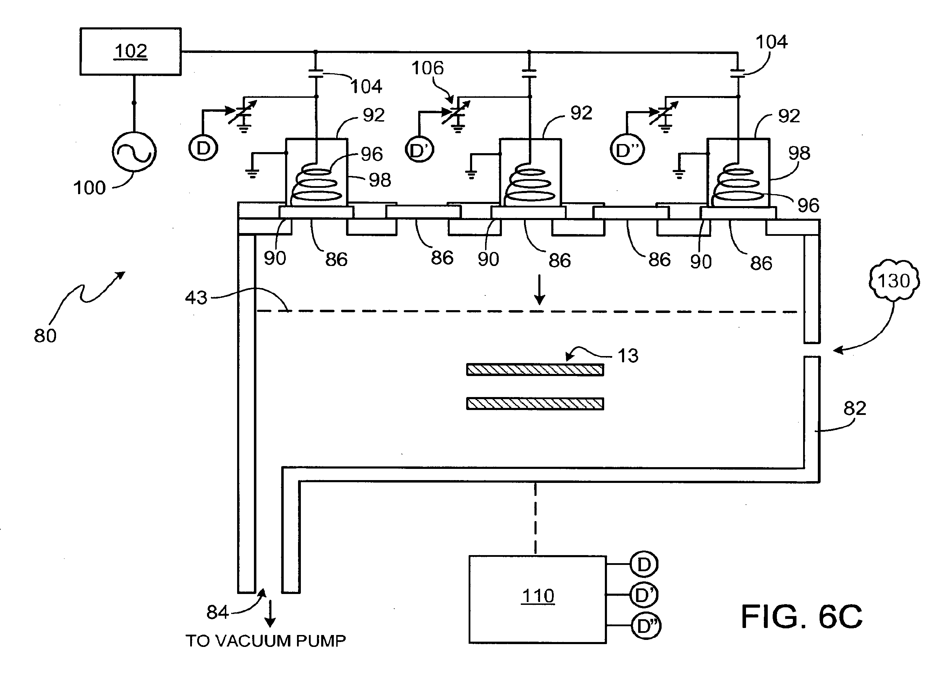

Referring to Fig. 6C an embodiment of a PIII processing system 80 includes a vacuum chamber 82 having a vacuum port 84 connected to a vacuum pump and a metal plasma source 130 for delivering a metal ion, e.g., aluminum ion, to chamber 82. System 80 includes a series of dielectric windows 86, e.g., made of glass or quartz, sealed by o-rings 90 to maintain a vacuum in chamber 82. Removably attached to some of the windows 86 are RF plasma sources 92, each source having a helical antenna 96 located within a grounded shield 98. The windows without attached RF plasma sources are usable, e.g., as viewing ports into chamber 82. Each antenna 96 electrically communicates with an RF generator 100 through a network 102 and a coupling capacitor 104. Each antenna 96 also electrically communicates with a tuning capacitor 106. Each tuning capacitor 106 is controlled by a signal D, D', D" from a controller 110. By adjusting each tuning capacitor 106, the output power from each RF antenna 96 can be adjusted to maintain homogeneity of the generated plasma. The regions of the stent directly exposed to ions from the plasma can be controlled by rotating the stents about their axis. The stents can be rotated continuously during treatment to enhance a homogenous modification of the entire stent. Alternatively, rotation can be intermittent, or selected regions can be masked, e.g., with a polymeric coating, to exclude treatment of those masked regions. Stent can be implanted on only the abluminal surface by masking the inner stent lumen by mounting the stent on a metal rod. Alloy (or non-alloy composite) composition and depth of implanted surface region can be controlled by selecting the ion type, dosage per area, and substrate temperature, pulsing of the bombardment and kinetic energy. The substrate temperature is preferably 0.4 times or less of the melting temperature of the substrate temperature in Kelvin. The pulsing can be used to control substrate temperature to reduce or avoid overheating and weakening the metal substrate. For example, overheating can be avoided by using a pulse regime in which the continuous "ON" pulsing is replaced by several shorter "ON/OFF" cycles. Besides metal plasma, suitable plasma gases include nitrogen, argon, helium, hydrogen, oxygen, and xenon In particular embodiments, for forming an aluminum alloy surface on stainless steel, the plasma gas is nitrogen, the ion energy is about 10 keV - 100 keV, and the ion dosage about 10¹⁰-10¹² cm⁻³. Additional details of PIII and MPIIID are described by Chu, U.S. Patent No. 6,120,260; Brukner, Surface and Coatings Technology, 103-104, 227-230 (1998); Kutsenko, Acta Materialia, 52, 4329-4335 (2004); Guenzel, Surface & Coatings Technology, 136, 47-50, (2001); Guenzel, J. Vacuum Science & Tech. B, 17(2), 895-899, (1999), Anders, Surface and Coatings Technology, 93, 158-167 (1997), and Liu et al., Review of Scientific Instruments, 70, 1816-1820 (1999), Yenkov, Surface & Coatings Technology 201 (2007) 6752-58, Russi, Brazilian Journal of Physics, 34(4B), December 2004, 155. the entire disclosure of each of which is incorporated by reference herein. PIII is also discussed in U.S. Application No. 11/355,392, filed February 16, 2006 (U.S. Patent Application Publication No. 2007-0191923), and U.S. Application No. 11/355,368, filed February 16, 2006 (U.S. Patent Application Publication No. 2007-0191931). Forming alloy or non-alloy composite can also be achieved by embedding or implanting metal particles into stent surface using laser surface treatment, detailed description of which is disclosed in U.S. Application No. 11/934,362, filed November 2, 2007 [Attorney Docket No. 10527-804001].

Referring particularly to steps 502 and 503 in FIG. 5, the alloy (or the non-alloy composite) so formed is then dealloyed (or selectively etched) to form the porous region which is then loaded with a drug by, e.g., an electropolymerization process as discussed above. In some embodiments, the drug is loaded without any polymer or monomer, and loading can be facilitated by using a potential difference (e.g., a few tens of millivolts to a few volts) between the porous region and the drug. In some embodiments, the drug (or a composition including the drug and a polymer) is loaded into porous region by dip coating or spraying the stent in a drug saturated solvent (or a solution of the composition) and drying under low temperature, e.g. ambient conditions. The drug (or composition) is as a result precipitated into the porous region. The loading can be facilitated by repeatedly dipping and drying while the stent substrate is cooled under evacuated conditions. In embodiments, loading can also be facilitated by treating the porous region by corona discharge to make the surface more lipophilic, which attracts more lipophilic drugs to the surface. In other embodiments, the drug is applied to the porous region by a vapor deposition process, such as pulsed laser deposition. The drug can be deposited by providing drug as a target material in the PLD apparatus. In embodiments, about 25% or more, e.g. about 50 to 90% of the void volume of the porous region is occupied by drug (or the composition) after loading.

In some embodiments, steps 502 and 503 may be carried out simultaneously if proper electrolyte suitable for both dealloying and electropolymerization is used, such as ionic liquids. Suitable ionic liquids along with methods of using ionic liquids as electrolytes in the process of electropolymerization and selective dissolution of metal are disclosed in, e.g., Pang et al., Electrochimica Acta, 52, 6172-6177 (2007), Lu et al., Journal of The Electrochemical Society, 151, H33-H39 (2004), Murray et al., Electrochimica Acta, 51, 2471-2476 (2006), and Abbott et al., Electrochimica Acta, 51, 4420-4425 (2006), Gelinski, Electrochimica Acta 51 (2006) 5567-5580, Johnson, Electrochemical Society Interface, Spring 2007, p. 38.

The terms "therapeutic agent", "pharmaceutically active agent", "pharmaceutically active material", "pharmaceutically active ingredient", "drug," and other related terms may be used interchangeably herein and include, but are not limited to, small organic molecules, peptides, oligopeptides, proteins, nucleic acids, oligonucleotides, genetic therapeutic agents, non-genetic therapeutic agents, vectors for delivery of genetic therapeutic agents, cells, and therapeutic agents identified as candidates for vascular treatment regimens, for example, as agents that reduce or inhibit restenosis. By small organic molecule is meant an organic molecule having 50 or fewer carbon atoms, and fewer than 100 non-hydrogen atoms in total.

Exemplary therapeutic agents include, e.g., anti-thrombogenic agents (e.g., heparin); anti-proliferative/anti-mitotic agents (e.g., paclitaxel, 5-fluorouracil, cisplatin, vinblastine, vincristine, inhibitors of smooth muscle cell proliferation (e.g., monoclonal antibodies), and thymidine kinase inhibitors); antioxidants; anti-inflammatory agents (e.g., dexamethasone, prednisolone, corticosterone); anesthetic agents (e.g., lidocaine, bupivacaine and ropivacaine); anti-coagulants; antibiotics (e.g., erythromycin, triclosan, cephalosporins, and aminoglycosides); agents that stimulate endothelial cell growth and/or attachment. Therapeutic agents can be nonionic, or they can be anionic and/or cationic in nature. Therapeutic agents can be used singularly, or in combination. Preferred therapeutic agents include inhibitors of restenosis (e.g., paclitaxel), antiproliferative agents (e.g., cisplatin), and antibiotics (e.g., erythromycin). Additional examples of therapeutic agents are described in U.S. Patent Application Publication No. 2005/0216074. Polymers for drug elution coatings are also disclosed in U.S. Patent Application Publication No. 2005/019265A. A functional molecule, e.g. an organic, drug, polymer, protein, DNA, and similar material can be incorporated into groves, pits, void spaces, and other features of the stent.

Any stent described herein can be dyed or rendered radiopaque by addition of, e.g., radiopaque materials such as barium sulfate, platinum or gold, or by coating with a radiopaque material. The stent can include (e.g., be manufactured from) metallic materials, such as stainless steel (e.g., 316L, BioDur® 108 (UNS S29108), and 304L stainless steel, and an alloy including stainless steel and 5-60% by weight of one or more radiopaque elements (e.g., Pt, Ir, Au, W) (PERSS®) as described in US-2003-0018380-A1, US-2002-0144757-A1, and US-2003-0077200-A1), Nitinol (a nickel-titanium alloy), cobalt alloys such as Elgiloy, L605 alloys, MP35N, titanium, titanium alloys (e.g., Ti-6A1-4V, Ti-50Ta, Ti-10Ir), platinum, platinum alloys, niobium, niobium alloys (e.g., Nb-1Zr) Co-28Cr-6Mo, tantalum, and tantalum alloys. Other examples of materials are described in commonly assigned U.S. Application No. 10/672,891, filed September 26, 2003 (U.S. Patent Application Publication No. 2005-0070990); and U.S. Application No. 11/035,316, filed January 3, 2005 (U.S. Patent Application Publication No. 2006-0153729). Other materials include elastic biocompatible metal such as a superelastic or pseudo-elastic metal alloy, as described, for example, in Schetsky, L. McDonald, "Shape Memory Alloys", Encyclopedia of Chemical Technology (3rd ed.), John Wiley & Sons, 1982, vol. 20. pp. 726-736; and commonly assigned U.S. Application No. 10/346,487, filed January 17, 2003 (U.S. Patent Application Publication No. 2004-0143317).

The stents described herein can be configured for vascular, e.g. coronary and peripheral vasculature or non-vascular lumens. For example, they can be configured for use in the esophagus or the prostate. Other lumens include biliary lumens, hepatic lumens, pancreatic lumens, urethral lumens.

The stent can be of a desired shape and size (e.g., coronary stents, aortic stents, peripheral vascular stents, gastrointestinal stents, urology stents, tracheal/bronchial stents, and neurology stents). Depending on the application, the stent can have a diameter of between, e.g., about 1 mm to about 46 mm. In certain embodiments, a coronary stent can have an expanded diameter of from about 2 mm to about 6 mm. In some embodiments, a peripheral stent can have an expanded diameter of from about 4 mm to about 24 mm. In certain embodiments, a gastrointestinal and/or urology stent can have an expanded diameter of from about 6 mm to about 30 mm. In some embodiments, a neurology stent can have an expanded diameter of from about 1 mm to about 12 mm. An abdominal aortic aneurysm (AAA) stent and a thoracic aortic aneurysm (TAA) stent can have a diameter from about 20 mm to about 46 mm. The stent can be balloon-expandable, self-expandable, or a combination of both (e.g., U.S. Patent No. 6,290,721).

The processes can be performed on other endoprostheses or medical devices, such as a stent precursor, e.g. metal tube, catheters, guide wires, and filters.

## Claims

1. A method of forming an endoprosthesis, wherein the endoprosthesis is one of a stent, stent precursor, catheter, guide wire or filter, comprising:
forming a porous metal surface (27) on the endoprosthesis,
introducing a drug (37, 42) by entrapping the drug (37, 42) in a polymer matrix (35, 41),
wherein the polymer is formed by electropolymerizing, and
wherein the drug (37, 42) is introduced into the porous metal surface (27) by applying an electrical potential to the metal surface.

2. The method of claim 1 wherein the electrical potential applied to the metal surface is positive and the monomer is electrochemically oxidized.

3. The method of claim 1 wherein the electrical potential applied to the metal surface (27) is positive and the drug (37, 42) has a negative charge.

4. The method of claim 1 wherein the electrical potential applied to the metal surface (27) is negative and the drug (37, 42) has a positive charge.

5. The method of claim 1 comprising forming the porous metal surface (27) by dealloying the surface of the endoprosthesis body.

6. The method of claim 1 comprising forming the porous metal surface (27) by etching.

7. The method of claim 1 further comprising forming an alloy layer on the endoprosthesis before forming the porous metal surface (27).

8. The method of claim 7 comprising forming the alloy layer by an implantation process.

9. The method of claim 8 comprising forming the porous metal surface (27) by dealloying the alloy layer.

## Patentansprüche

1. Verfahren zum Bilden einer Endoprothese, wobei die Endoprothese eines ist aus einem Stent, einem Stent-Precursor, Katheter, Führungsdraht oder Filter, umfassend:
Bilden einer porösen Metalloberfläche (27) auf der Endoprothese,
Einbringen eines Wirkstoffs (37, 42) durch Einschließen des Wirkstoffs (37, 42) in eine Polymermatrix (35, 41),
wobei das Polymer durch Elektropolymerisation gebildet wird, und
wobei der Wirkstoff (37, 42) in die poröse Metalloberfläche (27) durch Anlegen eines elektrischen Potentials an die Metalloberfläche eingeführt wird.

2. Verfahren nach Anspruch 1, wobei das elektrische Potential, das an die Metalloberfläche angelegt wird positiv ist und das Monomer elektrochemisch oxidiert wird.

3. Verfahren nach Anspruch 1, wobei das elektrische Potential, das an die Metalloberfläche (27) angelegt wird positiv ist und der Wirkstoff (37, 42) eine negative Ladung hat.

4. Verfahren nach Anspruch 1, wobei das elektrische Potential, das an die Metalloberfläche (27) angelegt wird negativ ist und der Wirkstoff (37, 42) eine positive Ladung hat.

5. Verfahren nach Anspruch 1 umfassend Bilden der porösen Metalloberfläche (27) durch Ablegieren der Oberfläche des Endoprothese-Körpers.

6. Verfahren nach Anspruch 1 umfassend Bilden der porösen Metalloberfläche (27) durch Ätzen.

7. Verfahren nach Anspruch 1 weiter umfassend Bilden einer Legierungsschicht auf der Endoprothese vor dem Bilden der porösen Metalloberfläche (27).

8. Verfahren nach Anspruch 7 umfassend Bilden der Legierungsschicht durch einen Implantationsprozess.

9. Verfahren nach Anspruch 8 umfassend Bilden der porösen Metalloberfläche (27) durch Ablegieren der Legierungsschicht.

## Revendications

1. Procédé de formation d'une endoprothèse, dans lequel l'endoprothèse est un stent, un précurseur de stent, un cathéter, un fil-guide ou un filtre, comprenant :
la formation d'une surface métallique poreuse (27) sur l'endoprothèse,
l'introduction d'un médicament (37, 42) par piégeage du médicament (37, 42) dans une matrice polymère (35, 41),
dans lequel le polymère est formé par électropolymérisation, et
dans lequel le médicament (37, 42) est introduit dans la surface métallique poreuse (27) en appliquant un potentiel électrique à la surface métallique.

2. Procédé selon la revendication 1 dans lequel le potentiel électrique appliqué à la surface métallique est positif et le monomère est oxydé électrochimiquement.

3. Procédé selon la revendication 1 dans lequel le potentiel électrique appliqué à la surface métallique (27) est positif et le médicament (37, 42) porte une charge négative.

4. Procédé selon la revendication 1 dans lequel le potentiel électrique appliqué à la surface métallique (27) est négatif et le médicament (37, 42) porte une charge positive.

5. Procédé selon la revendication 1 comprenant la formation de la surface métallique poreuse (27) par corrosion sélective de la surface du corps d'endoprothèse.

6. Procédé selon la revendication 1 comprenant la formation de la surface métallique poreuse (27) par gravure.

7. Procédé selon la revendication 1 comprenant en outre la formation d'une couche d'alliage sur l'endoprothèse avant la formation de la surface métallique poreuse (27).

8. Procédé selon la revendication 7 comprenant la formation de la couche d'alliage par un procédé d'implantation.

9. Procédé selon la revendication 8 comprenant la formation de la surface métallique poreuse (27) par corrosion sélective de la couche d'alliage.
